(19) Europäisches Patentamt

European Patent Office

Office européen des brevets

(11) **EP 4 295 129 B1**

(12) **EUROPÄISCHE PATENTSCHRIFT**

(45) Veröffentlichungstag und Bekanntmachung des Hinweises auf die Patenterteilung:
**03.06.2026   Patentblatt 2026/23**

(21) Anmeldenummer: **22726085.8**

(22) Anmeldetag: **28.04.2022**

(51) Internationale Patentklassifikation (IPC):
*G01J 3/10* (2006.01)      *G01J 5/0875* (2022.01)
*G01J 5/20* (2006.01)      *G01K 7/16* (2006.01)
*G01J 3/02* (2006.01)      *A61B 18/18* (2006.01)
*A61F 7/00* (2006.01)      *G01K 1/14* (2021.01)

(52) Gemeinsame Patentklassifikation (CPC):
**G01J 3/108; A61F 7/00; A61F 7/007; G01J 5/0875; G01J 5/20; G01K 1/14;** A61F 2007/0086; A61F 2007/0088; G01J 3/0256

(86) Internationale Anmeldenummer:
**PCT/EP2022/061320**

(87) Internationale Veröffentlichungsnummer:
**WO 2022/229313 (03.11.2022 Gazette 2022/44)**

(54) **THERMISCHE STRAHLUNGSQUELLE UND VERFAHREN ZUR MESSUNG DER EXAKTEN TEMPERATUR UND / ODER ABGESTRAHLTEN STRAHLUNGSLEISTUNG DER THERMISCHEN STRAHLUNGSQUELLE**

THERMAL RADIATION SOURCE AND PROCEDURE FOR MEASURING THE EXACT TEMPERATURE AND/OR RADIATED POWER OF THE THERMAL RADIATION SOURCE

SOURCE DE RAYONNEMENT THERMIQUE ET PROCÉDÉ DE MESURE DE LA TEMPÉRATURE EXACTE ET / OU DE LA PUISSANCE RAYONNÉE DE LA SOURCE DE RAYONNEMENT THERMIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.04.2021   DE 102021111260**

(43) Veröffentlichungstag der Anmeldung:
**27.12.2023   Patentblatt 2023/52**

(73) Patentinhaber: **Infrasolid GmbH**
**01217 Dresden (DE)**

(72) Erfinder: **SCHOSSIG, Marco**
**01189 Dresden (DE)**

(74) Vertreter: **Querner, Yvonne Anne**
**Chemnitzer Straße 105**
**01187 Dresden (DE)**

(56) Entgegenhaltungen:
| | |
|---|---|
| EP-A1- 1 789 760 | CN-A- 112 261 749 |
| DE-A1- 102004 053 012 | DE-A1- 102018 101 974 |
| DE-A1- 102019 109 842 | US-A1- 2004 133 084 |
| US-A1- 2016 361 449 | US-A1- 2019 246 457 |

**Beschreibung**

Die Erfindung betrifft eine thermische Strahlungsquelle, die die Merkmale nach Anspruch 1 umfasst.

Die Erfindung umfasst ebenfalls zwei Verfahren zur Messung der Temperatur eines Austrittsfensters der erfindungsgemäßen thermischen Strahlungsquelle und der Strahlungsleistung einer emittierten Strahlung der erfindungsgemäßen thermischen Strahlungsquelle durch ein Austrittsfenster, die jeweils durch die Merkmale der Ansprüche 17 und 18 definiert werden.

Thermische Strahlungsquellen finden vor allem in der Analyse- und Messtechnik Anwendung, um z. B. die Konzentration von Stoffen (Gase, Flüssigkeiten, Feststoffe) zu detektieren. Ein weiteres großes Einsatzgebiet ist die Medizintechnik zur Behandlung und Linderung von Krankheiten und Verletzungen. In allen Anwendungsfällen ist eine genaue Kenntnis über die ausgesandte Strahlungsleistung bzw. Strahlungsdosis des Strahlungselementes der thermischen Strahlungsquelle notwendig, um exakte, reproduzierbare und langzeitstabile Messungen bzw. definierte Behandlungen durchführen zu können. Die Strahlungsleistung $P_S$ einer thermischen Strahlungsquelle hängt im Wesentlichen von seiner Temperatur $T$ ab und wird durch das Stefan-Boltzmann-Gesetz beschrieben:

$$P_S = \sigma \cdot \varepsilon \cdot A \cdot T^4$$

mit der Stefan-Boltzmann-Konstante $\sigma$ sowie dem Emissionsgrad $\varepsilon$ und der Fläche $A$ der Strahlungsquelle.

Die Wellenlänge $\lambda_{\max}$, bei der das Maximum der Ausstrahlung liegt und wodurch die spektrale Verteilung bestimmt wird, hängt ebenfalls von der Temperatur $T$ der Strahlungsquelle ab und wird durch das Wiensche Verschiebungsgesetz beschrieben:

$$\lambda_{max} = \frac{2897{,}8 \ \mu m \cdot K}{T}.$$

Überdies ist es vor allem bei medizinischen und therapeutischen Anwendungen von großer Bedeutung, neben der Strahlungsdosis auch die Temperatur des Austrittsfensters zu kennen, damit es bei direktem Hautkontakt nicht zu einer Verbrennung der Haut kommen kann.

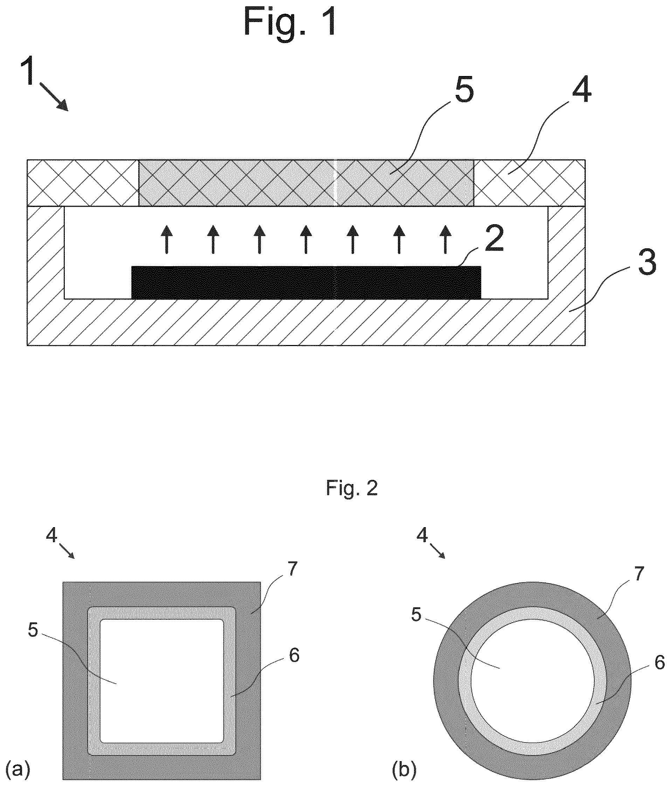

Eine thermische Strahlungsquelle 1 besteht im Wesentlichen aus einem elektrisch betriebenen Strahlungselement 2, z. B. einem Heizleiterelement, das in einem Gehäuse 3 angeordnet ist, wobei die Strahlung des Strahlungselements durch ein Austrittsfenster 4 aus dem Gehäuse 3 ausgesandt wird (Fig. 1). Das Strahlungs- oder Heizleiterelement 2 wird dabei typischerweise auf eine Temperatur > 500 °C erwärmt, um ein breites Emissionsspektrum im infraroten Spektralbereich zu erzeugen. Für Anwendungen im sichtbaren und nahinfraroten Bereich sind deutlich höhere Temperaturen von > 1000 °C notwendig. D. h. die Temperatur ist je nach Anwendung und Einsatzbereich regelbar, um ein gewünschtes Emissionsspektrum mit der thermischen Strahlungsquelle zu erzielen.

Aus dem Stand der Technik sind Silizium-basierte MEMS-Bauelemente mit integrierten Temperatursensoren bekannt, z. B. aus der US 5,391,875 A, womit die Temperatur des Si-Chips gemessen werden kann. In der US 8,859,303 B2 ist dies beispielsweise für eine thermische Infrarot-Strahlungsquelle beschrieben. Mit Hilfe des integrierten Temperatursensors wird die Temperatur der Strahlungsquelle gemessen, wodurch eine Regelung der Temperatur der Strahlungsquelle möglich ist. Über die Zusammenhänge der Strahlungsgesetze wird damit die ausgesandte Strahlungsleistung abgeschätzt und versucht konstant zu halten. Allerdings ist damit keine exakte Bestimmung der tatsächlich aus dem Gehäuse der Strahlungsquelle ausgesandten Strahlungsleistung möglich sowie auch keine Überwachung von Alterungserscheinungen der Strahlungsquelle. Zum Beispiel hat die räumliche Abstrahlcharakteristik von thermischen Strahlungsquellen zur Folge, dass ein Teil der emittierten Strahlung im Gehäuse gefangen bleibt. Ebenso führen Änderungen des Emissionsgrads, z. B. durch Oxidation oder Alterung, zu einer Änderung der Strahlungsleistung.

Aus der US 2016/0361449 A1 ist eine Elektronenstrahl-Sterilisationsvorrichtung zum Sterilisieren eines Verpackungsbehälters durch Bestrahlung mit Elektronen aus einer Elektronenstrahlquelle bekannt. Für eine zuverlässige Sterilisation muss eine Mindestelektronenenergie bzw. Strahlintensität der Elektronen der Elektronenstrahlquelle gewährleistet werden, wobei die emittierten Elektronen am Austrittsfenster der Vorrichtung Temperaturen im Bereich von 300°C bis 400°C erzeugen. Die abgegebene Energie bzw. Strahlintensität der Elektronen aus der Elektronenstrahlquelle wird über eine Messung der Temperatur innerhalb des Gehäuses oder am Austrittsfenster der Vorrichtung bestimmt. Nachteilig ist die aufwendige Konstruktion des Austrittsfensters aus Kupferrippen und einer die Kupferrippen überspannenden Metallfolie mit einer Durchlässigkeit für Elektronen, aber nicht für elektromagnetische Strahlung. Die für die Messung der Temperatur verwendeten Thermoelemente sind auf den Kupferrippen oder der Metallfolie angeordnet und mit einem Material bedeckt, welches geeignet ist, Wärme zu erzeugen, wenn es mit Elektronen beaufschlagt wird. Die von den Thermoelementen detektierte Temperatur wird für die Bestimmung der Strahlintensität der emittierten Elektronen der Elektronenstrahlquelle benutzt, d.h. die Thermoelemente müssen im Elektronenstrahl angeordnet sein. Aufgrund der höheren Wärmekapazität der verwendeten Thermoelemente im Vergleich zur Wärmekapazität der nur 4 bis 12 $\mu m$ dicken Metallfolie erfolgt keine direkte Messung der Temperatur des Austrittsfensters. Die Strahlungsintensität einer thermischen Strahlungsquelle kann mit der Vorrichtung aus der US 2016/0361449 A1 nicht gemessen werden, da die thermische Strahlung an dem (metallischen) Austrittsfenster reflektiert werden würde.

In der DE 198 07 453 A1 wird eine Vorrichtung zur Erzeugung von Wärmestrahlung für medizinische und therapeutische Anwendungen, wie z. B. die Heilung von Insektenstichen, beschrieben. Eine Verbrennung der Haut wird hier durch einen verschiebbaren Schutzmantel zur Aufrechterhaltung eines Sicherheitsabstands erreicht. Eine Messung der Strahlungsleistung bzw. Strahlungsdosis und / oder der Temperatur der Kontaktfläche, d. h. eines Austrittsfensters, erfolgt nicht. Eine optimale und dosierte Behandlung ist mit diesem Aufbau nicht möglich. Zudem besteht eine Verbrennungsgefahr, wenn der Sicherheitsabstand falsch eingestellt ist. Eine ähnliche jedoch miniaturisierte Wärmeübertragungseinrichtung zur berührungslosen thermischen Behandlung ist in der DE 10 2019 109 842 A1 offenbart, jedoch ohne die Möglichkeit die Temperatur am Austrittsfenster zu bestimmten.

US2004133084 A1 offenbart eine Messvorrichtung im Infrarotbereich für die Bestimmung von Analyten in Gewebe/Flüssigkeiten, sowohl nicht-invasiv (Haut) als auch in vitro. Die Vorrichtung umfasst eine Fensterbaugruppe aus CVD-Diamant, eine darauf angeordnete Heizschicht mit Leiterbahnen als Heizelement und integrierte Widerstandsthermometer in der Heizschicht zur präzisen Temperaturregelung des Fensters. Strahlungsdetektoren sind im optischen Weg hinter dem Fenster angebracht.

Zusammenfassend ist bis dato keine Vorrichtung für eine integrierte Lösung bekannt, die eine Messung der von einer thermischen Strahlungsquelle ausgesandten Strahlungsleistung bzw. Strahlungsdosis und die eine Messung der Oberflächentemperatur des Austrittsfenster einer thermischen Strahlungsquelle ermöglicht.

Es ist daher eine Aufgabe der vorliegenden Erfindung eine Möglichkeit zu schaffen, die von einer thermischen Strahlungsquelle die ausgesandte Strahlungsleistung bzw. Strahlungsdosis und die Oberflächentemperatur des Austrittsfensters einer solchen thermischen Strahlungsquelle messen kann.

Die Aufgabe wird durch eine thermische Strahlungsquelle gemäß dem unabhängigen Patentanspruch 1 gelöst.

Die erfindungsgemäße thermische Strahlungsquelle umfasst die Merkmale nach Anspruch 1. Weitere Ausführungsformen werden durch die abhängigen Ansprüche 2-16 definiert.

Unter einem homogenen Austrittsfenster wird ein Austrittsfenster verstanden, welches eine homogene Dicke im Bereich von $200\,\mu m$ bis 2mm aufweist, besteht und gegenüber einem verwendeten Temperatursensor und Strahlungssensor eine wesentlich höhere Wärmekapazität aufweist.

Der Vorteil gegenüber bisher bekannten Lösungen liegt insbesondere darin, dass die tatsächlich aus dem Gehäuse der Strahlungsquelle ausgesandte Strahlungsleistung bzw. die Temperatur des Austrittsfensters gemessen wird und somit geregelt und überwacht werden kann.

Dies wird insbesondere dadurch erreicht, dass das Austrittsfenster der erfindungsgemäßen thermischen Strahlungsquelle in drei verschiedene Funktionsbereiche gegliedert ist, einen Durchlassbereich, einen Kontaktierungsbereich und einen Messbereich. Der Durchlassbereich kennzeichnet den Funktionsbereich des Austrittsfensters, aus dem die von dem Strahlungselement der thermischen Strahlungsquelle emittierte Strahlung aus dem Gehäuse in die Umgebung abgestrahlt wird. Der Durchlassbereich sollte vorzugsweise der Größe des Strahlungselementes entsprechen. Der Messbereich kennzeichnet den Funktionsbereich des Austrittsfensters, innerhalb dem der Temperatursensor und der Strahlungssensor angeordnet sind. Der Kontaktierungsbereich kennzeichnet den Funktionsbereich des Austrittsfensters, in welchem die Kontaktierung von dem Temperatur- und dem Strahlungssensor erfolgt, wobei der Kontaktierungsbereich im Randbereich des Austrittsfensters ausgebildet ist, wo das Austrittsfenster in Kontakt mit dem Gehäuse der thermischen Strahlungsquelle steht. Das Austrittsfenster weist bei einer Betrachtung seines Querschnitts von innen nach außen den Durchlassbereich, einen daran anschließenden Messbereich und einen wiederum daran anschließenden Kontaktierungsbereich auf, wobei allerdings der Durchlassbereich und der Messbereich getrennt voneinander und teilweise überlappend ausgebildet sein können. Teilweise überlappend im Sinne der Erfindung bedeutet, dass der Messbereich auch im Durchlassbereich des Austrittsfensters ausgebildet sein kann, z. B. indem mittig im Durchlassbereich ein Temperatursensor und ein Strahlungssensor angeordnet sind, der über schmale Anschlussbahnen, die durch den Durchlassbereich zum Kontaktierungsbereich geführt sind, kontaktiert ist.

Das Material des Austrittsfensters ist erfindungsgemäß Saphir, da Saphir über einen weiten Wellenlängenbereich transparent, zudem sehr robust, chemisch inert und damit auch biokompatibel ist. Alternativ können aber auch andere infrarotdurchlässige Materialien, wie z. B. Silizium, Germanium, Kalziumfluorid, Bariumfluorid oder Zinkselenid, verwendet werden.

Als Temperatur- und Strahlungssensor im Messbereich des Austrittsfensters wird vorzugsweise ein Platin-Messwiderstand in Dünnschichttechnik eingesetzt, weil dieser einen nahezu temperaturlinearen Widerstandsverlauf und einen weiten Temperaturmessbereich aufweist sowie kostengünstig hergestellt werden kann. Grundsätzlich kann aber auch jeder andere Temperatur- oder Strahlungssensortyp eingesetzt werden, wie z. B. Halbleitersensoren oder Thermoelemente. Für die Kontaktierung der Temperatur- und Strahlungssensoren im Messbereich kann beispielsweise Indium-Zinn-Oxid (ITO) eingesetzt werden, wie es z. B. als transparente Dünnschicht-Leiterbahn bei OLEDs eingesetzt wird. Wird ITO als Material für die Leiterbahnen auf dem Austrittsfenster eingesetzt, kann der Messbereich in der Mitte (oder räumlich beliebig) auf dem Austrittsfenster angeordnet sein, d.h. teilweise überlappend mit dem Durchlassbereich. Wenn zudem der Temperatursensor und Strahlungssensor transparent für die Strahlung ist, dann können der Messbereich und der Durchlassbereich in einer Variante deckungsgleich ausgebildet sein.

Durch das Aufbringen eines Temperatur- und Strahlungssensors auf das Austrittsfenster der thermischen Strahlungsquelle kann u. a. die Qualität und die Zuverlässigkeit von Messungen, beispielsweise in der Stoffanalyse, verbessert werden. Unter dem Aufbringen eines Temperatur- und Strahlungssensors innerhalb des Messbereichs des Austrittsfensters wird im Sinne der Erfindung verstanden, dass der Temperatur- und Strahlungssensor, z. B. mittels Dünnschichttechnik oder Schablonen- / Sieb-Druck-Verfahren direkt auf die Oberfläche des Austrittsfensters aufgebracht wird oder auf dem Austrittsfenster, z. B. durch Kleben, aufgebracht wird bzw. angeordnet ist. Dadurch lassen sich auch Alterungserscheinungen der Strahlungsquelle besser überwachen. Bei medizinischen und therapeutischen Anwendungen lässt sich die Strahlungsdosis exakt messen und auf die Behandlung abstimmen. Durch die Messung der Oberflächentemperatur des Austrittsfensters lassen sich Verbrennungen bei Anwendungen mit Hautkontakt vermeiden bzw. werden dadurch Anwendungen mit Hautkontakt erst sinnvoll möglich. Das erhöht die Zuverlässigkeit der medizinischen Geräte und sorgt für eine optimale Behandlung. Bei Anwendungen in der Analytik und Messtechnik, wie z. B. in der Gasanalyse, lassen sich zudem temperaturabhängige spektrale Verschiebungen der Transmissionseigenschaften des Austrittsfensters, insbesondere an Filterkanten, bestimmen und quantifizieren und in der Messung korrigieren. Das erhöht die Messgenauigkeit und Zuverlässigkeit.

Besonders vorteilhaft ist die Kombination der erfindungsgemäßen thermischen Strahlungsquelle mit einem Gehäuse für die Oberflächenmontage, einem sogenannten SMD-Gehäuse (Surface Mount Device-Gehäuse), weil dadurch ein hochintegriertes Bauteil ohne externe Kontaktierung, eine sehr flache Bauweise und die Möglichkeit einer vollautomatisierten Fertigung erreicht werden.

In einer Ausgestaltung der erfindungsgemäßen thermischen Strahlungsquelle ist der Messbereich von dem Durchlassbereich und dem Kontaktierungsbereich umfasst ausgebildet. D. h. der Messbereich wird von dem Durchlassbereich und Kontaktierungsbereich eingegrenzt. Der oder die Temperatur- und Strahlungssensoren sind dabei im Messbereich angeordnet. Das hat den Vorteil, dass in unmittelbarer Umgebung des Durchlassbereichs des Austrittsfensters, dort wo die Strahlung hindurchtritt, die Temperatur bzw. die Strahlungsleistung direkt gemessen werden kann.

In einer anderen Ausgestaltung der erfindungsgemäßen thermischen Strahlungsquelle ist der Messbereich deckungsgleich mit dem Durchlassbereich ausgebildet. Das hat den Vorteil, dass die Temperatur bzw. die Strahlungsleistung direkt im Austrittsfenster, beispielsweise in der Mitte des Austrittsfensters, dort wo die Strahlung hindurchtritt, gemessen werden kann.

In einer weiteren Ausgestaltung der erfindungsgemäßen thermischen Strahlungsquelle weist das Austrittsfenster eine rechteckige, quadratische oder eine runde Form auf. Eine rechteckige oder quadratische Form des Austrittsfensters ist insbesondere für Gehäuse der Oberflächenmontage, sogenannte SMD-Gehäuse, aber auch eckige Gehäuseformen der Durchsteckmontage geeignet. Ein rundes Austrittsfenster wird vorzugsweise für runde Gehäuseformen, wie z. B. bei TO-Gehäusen, eingesetzt.

In einer bevorzugten Ausgestaltung der erfindungsgemäßen thermischen Strahlungsquelle ist der Temperatur- und Strahlungssensor deckungsgleich mit dem Messbereich des Austrittsfensters ausgebildet. Deckungsgleich bedeutet im Sinne der Erfindung, dass der Temperatursensor und Strahlungssensor im Messbereich des Austrittsfensters vorteilhafterweise umlaufend bzw. die gleiche Fläche wie der Messbereich aufweisend ausgebildet ist, so dass Temperatur und Strahlungsleistung möglichst exakt gemessen werden können, d. h. im gesamten Messbereich des Austrittsfensters ist mittels des Temperatur- und Strahlungssensors die Temperatur bzw. Strahlungsleistung detektierbar.

In einer anderen vorteilhaften Ausgestaltung der erfindungsgemäßen thermischen Strahlungsquelle ist mehr als ein Temperatur- und Strahlungssensor innerhalb des Messbereichs des Austrittsfensters angeordnet. Durch die Anordnung von mehr als einem Temperatur- und Strahlungssensors, vorzugsweise einer Vielzahl von Temperatur- und Strahlungssensoren, ist es möglich, die Temperatur und Strahlungsleistung ortsaufgelöst zu messen.

In einer anderen Ausgestaltung der erfindungsgemäßen thermischen Strahlungsquelle ist der im Messbereich des Austrittsfensters aufgebrachte Temperatur- und Strahlungssensor innerhalb des Gehäuses angeordnet, wobei eine elektrische Drahtkontaktierung des Temperatur- und Strahlungssensors ebenfalls innerhalb des Gehäuses ausgebildet ist. Eine innenliegende Anordnung des Temperatur- und Strahlungssensors im Messbereich des Austrittsfensters und deren Drahtkontaktierung im Kontaktierungsbereich hat u. a. den Vorteil, dass alle Komponenten vor externen Einflüssen, z. B. Beschädigungen, gut geschützt sind. Obwohl der Temperatursensor bei dieser Anordnung nicht direkt die Oberflächentemperatur der außenliegenden, einem (Mess-) Objekt zugewandten Seite des Austrittsfensters misst und der Strahlungssensor nicht direkt die von der thermischen Strahlungsquelle ausgesandte Strahlungsleistung, die durch das Austrittsfenster tritt, erfasst, kann beides durch eine Kalibrierung des Systems und durch Kenntnis der Materialeigenschaften, wie z. B. der Transmission und Wärmeleitfähigkeit des Austrittsfensters, in der Signalverarbeitung des Systems korrigiert werden.

In einer weiteren Ausgestaltung der erfindungsgemäßen thermischen Strahlungsquelle ist der im Messbereich des Austrittsfensters angeordnete Temperatur- und Strahlungssensor außerhalb des Gehäuses angeordnet, wobei eine elektrische Drahtkontaktierung des Temperatur- und Strahlungssensors ebenfalls außerhalb des Gehäuses ausgebildet ist. In dieser Anordnung wird vorteilhafterweise direkt die von der Strahlungsquelle ausgesandte Strahlungsleistung, die durch das Austrittsfenster tritt, erfasst und auch die Oberflächentemperatur der einem (Mess-) Objekt zugewandten Seite des Austrittsfensters gemessen. Der Temperatur- und Strahlungssensor im Messbereich des Austrittsfensters und deren Kontaktdrähte können durch das Aufbringen einer zusätzlichen dünnen Passivierungs- und Schutzschicht, aus z. B. Glas, vor äußeren Einflüssen geschützt werden.

In einer anderen weiteren Ausgestaltung der erfindungsgemäßen thermischen Strahlungsquelle ist der im Messbereich des Austrittsfensters aufgebrachte Temperatur- und Strahlungssensor innerhalb des Gehäuses angeordnet, wobei eine elektrische Drahtkontaktierung des Temperatur- und Strahlungssensors außerhalb des Gehäuses ausgebildet ist. Wenn bei einer Anordnung des Temperatur- und Strahlungssensors im Messbereich des Austrittsfensters innerhalb des Gehäuses kein Platz für Kontaktdrähte vorhanden oder dies technologisch nicht kompatibel ist, kann eine außenliegende Drahtkontaktierung derart erfolgen, dass das Austrittsfenster über das Gehäuse hinausragt und der Kontaktierungsbereich ebenfalls bis nach außen reicht, so dass die Kontaktierung außerhalb des Gehäuses liegt.

In einer besonders bevorzugten Ausgestaltung der erfindungsgemäßen thermischen Strahlungsquelle ist eine Kontaktierung des Temperatur- und Strahlungssensors über außerhalb an dem Gehäuse angebrachte Kontaktflächen mittels Löten einer metallischen Lotpaste und / oder mittels leitfähigem Klebstoff ausgebildet. Mit dieser Variante ist die Realisierung eines hochintegrierten und langzeitstabilen Bauteils für die automatisierte Herstellung in hohen Stückzahlen möglich, wobei besonders die drahtlose Kontaktierung von Temperatur- und Strahlungssensor, z. B. durch Kleben mit einem leitfähigen Klebstoff oder Löten mit einer metallischen Lotpaste, sowie ein Gehäuse für die Oberflächenmontage vorteilhaft sind. Die elektrische Kontaktierung des Temperatursensors und Strahlungssensors erfolgt dabei über passende Kontaktflächen auf dem Gehäuse für die Oberflächenmontage.

In einer weiteren bevorzugten Ausgestaltung der erfindungsgemäßen thermischen Strahlungsquelle ist der im Messbereich des Austrittsfensters aufgebrachte Temperatur- und Strahlungssensor außerhalb des Gehäuses angeordnet, wobei eine elektrische Kontaktierung des Temperatur- und Strahlungssensors über Kontaktflächen, die auf einer Halterung, die mit der thermischen Strahlungsquelle verbunden ist, ausgebildet ist. Die thermische Strahlungsquelle ist an einer Halterung oder Einbauvorrichtung mit lötbaren Kontaktflächen befestigt und mittels Lotpaste oder leitfähigem Klebstoff im Kontaktierungsbereich elektrisch kontaktiert. Die Halterung kann beispielsweise eine Leiterplatte oder ein anderes Gehäuse oder etwas ähnliches sein. Dies hat den Vorteil, dass die elektrischen Kontakte vor Beschädigung und anderen äußeren Umwelteinflüssen geschützt sind und nur der Messbereich des Austrittsfensters mit einer Passivierungs- und Schutzschicht abgedeckt werden müsste.

In einer anderen Ausgestaltung der erfindungsgemäßen thermischen Strahlungsquelle ist sowohl ein Temperatur- und Strahlungssensor innerhalb des Gehäuses im Messbereich des Austrittsfensters aufgebracht angeordnet als auch ein weiterer Temperatur- und Strahlungssensor außerhalb des Gehäuses im Messbereich des Austrittsfensters aufgebracht angeordnet, wobei eine elektrische Kontaktierung der Temperatur- und Strahlungssensoren mittels einer Drahtkontaktierung und/oder über außerhalb an dem Gehäuse angebrachte Kontaktflächen mittels Löten einer metallischen Lotpaste und / oder mittels leitfähigem Klebstoff ausgebildet ist. Die beidseitige Anordnung bzw. Integration von Temperatur- und Strahlungssensoren in dem Austrittsfenster, also zum einen auf der nach innen in das Gehäuse weisenden Fläche des Austrittsfensters als auch zum anderen auf der nach außen aus dem Gehäuse weisenden Fläche des Austrittsfensters, hat den Vorteil, dass sich Temperaturunterschiede zwischen Innen- und Außenseite des Austrittsfensters messen lassen, wodurch z. B. die Messgenauigkeit erhöht wird. Außerdem ist es damit möglich eine Vielzahl von Sensorelementen auf dem Austrittsfenster unterzubringen, um beispielsweise die Temperatur und die Strahlungsleistung ortsaufgelöst messen zu können.

In einer Variante der erfindungsgemäßen thermischen Strahlungsquelle ist das Austrittsfenster aus Silizium oder Germanium ausgebildet, wobei der Temperatur- und Strahlungssensor in diesem Fall im Messbereich des Austrittsfensters integriert angeordnet ist. Integriert bedeutet in diesem Sinne, dass der Temperatur- und Strahlungssensor mittels Verfahren der Halbleitertechnologie in der Ebene des Austrittsfensters ausgebildet, also integriert, wird.

In einer weiteren bevorzugten Ausgestaltung der erfindungsgemäßen thermischen Strahlungsquelle ist das elektrische Strahlungselement freitragend ausgebildet, wobei vorzugsweise unterhalb des Strahlungselementes eine Reflexionsschicht ausgebildet ist. Durch einen freitragenden Aufbau weist das Strahlungselement eine sehr gute thermische Isolation auf. In Kombination mit einer sehr geringen thermischen Masse des Strahlungselementes, wodurch es gut elektrisch modulierbar ist, kann ein maximaler Wirkungsgrad erreicht werden. Die Reflexionsschicht dient dazu die rückseitig emittierte Strahlung zu reflektieren und durch das Austrittsfenster zu leiten.

Die Aufgabe wird ebenfalls durch ein Verfahren gemäß dem unabhängigen Patentanspruch 17 gelöst.

Das erfindungsgemäße Verfahren ist durch die Merkmale des Anspruch 17 definiert.

Im ersten Schritt kann durch den Temperatursensor, der keine Strahlung detektiert, d. h. sieht, die Temperatur gemessen werden. Der Strahlungssensor detektiert die emittierte Strahlung kann aber auch gleichzeitig die Temperatur des Austrittsfensters messen. Um aus den beiden Sensorsignalen, die entweder durch den Temperatursensor und den Strahlungssensor oder nur durch den Strahlungssensor erfasst werden, die Strahlungsleistung zu ermitteln, ist eine Kalibrierung der thermischen Strahlungsquelle, wie nachfolgend beschrieben wird, zwingend erforderlich. Die Kalibrierung erfolgt einmalig nach der Herstellung des Bauelements, sie ist also nicht vor jeder Messung notwendig. Die Bestimmung bzw. Auswertung der Strahlungsleistung erfolgt durch eine mathematische Verknüpfung der aufgenommenen Sensorsignale. Die mathematische Verknüpfung kann beispielsweise eine Differenzbildung sein.

Die Aufgabe wird ebenso durch ein Verfahren gemäß dem unabhängigen Patentanspruch 18 gelöst.

Bei dem erfindungsgemäßen Verfahren ist durch die Merkmale des Anspruch 18 definiert.

Die Kalibrierung umfasst zum einen das Aufnehmen der Sensorsignale bei definierten Temperaturwerten und ausgeschaltetem Strahlungselement und zum anderen eine externe berührungslose Temperaturmessung des Austrittsfensters bei eingeschaltetem Strahlungselement und definierten Leistungskennwerten. Zusätzlich kann noch eine externe Messung der ausgesandten Strahlungsleistung bei definierten Leistungskennwerten des Strahlungselements erfolgen.

Die erfindungsgemäße thermische Strahlungsquelle ist insbesondere für den Einsatz in Messgeräten für die Stoff- und Spektralanalyse sowie medizinische und therapeutische Geräte zur Behandlung von Krankheiten und Verletzungen vorgesehen und dafür geeignet und ausgebildet.

Im Folgenden soll die Erfindung anhand von Ausführungsbeispielen näher erläutert werden.

Die zugehörigen Zeichnungen zeigen

Fig. 1 Prinzipieller Aufbau einer thermischen Strahlungsquelle nach dem Stand der Technik;

Fig. 2 Bevorzugte Ausgestaltungen des Austrittsfensters der erfindungsgemäßen thermischen Strahlungsquelle: (a) rechteckige/quadratische Form und (b) runde Form;

Fig. 3 Beispielhafte Ausführungsformen der erfindungsgemäßen thermischen Strahlungsquelle mit im Messbereich des Austrittsfensters aufgebrachtem Temperatursensor und Strahlungssensor:

(a) innenliegende Anordnung des Temperatursensors und Strahlungssensors sowie deren Drahtkontaktierung,
(b) außenliegende Anordnung des Temperatursensors und Strahlungssensors sowie deren Drahtkontaktierung sowie
(c) innenliegende Anordnung des Temperatursensors und Strahlungssensors mit außenliegender Drahtkontaktierung des Temperatursensors und Strahlungssensors;

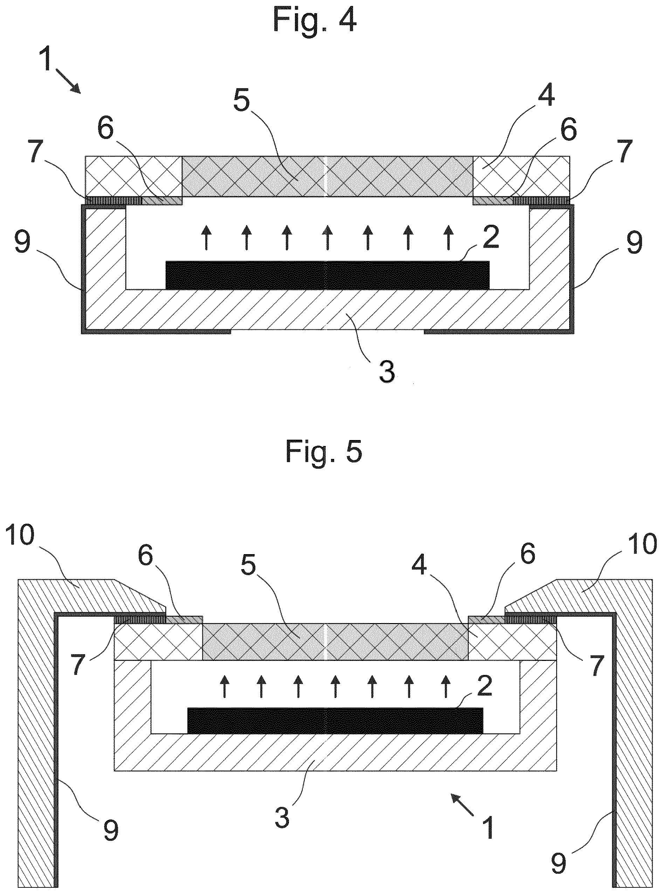

Fig. 4 Bevorzugte Ausführungsform der erfindungsgemäßen thermischen Strahlungsquelle mit drahtloser Kontaktierung des Temperatursensors und Strahlungssensors über Kontaktflächen in einem Gehäuse für die Oberflächenmontage;

Fig. 5 Ausführungsform der erfindungsgemäßen thermischen Strahlungsquelle mit außenliegender Anordnung des Temperatursensors und Strahlungssensors sowie einer Kontaktierung über Kontaktflächen, die auf einer Halterung ausgebildet sind;

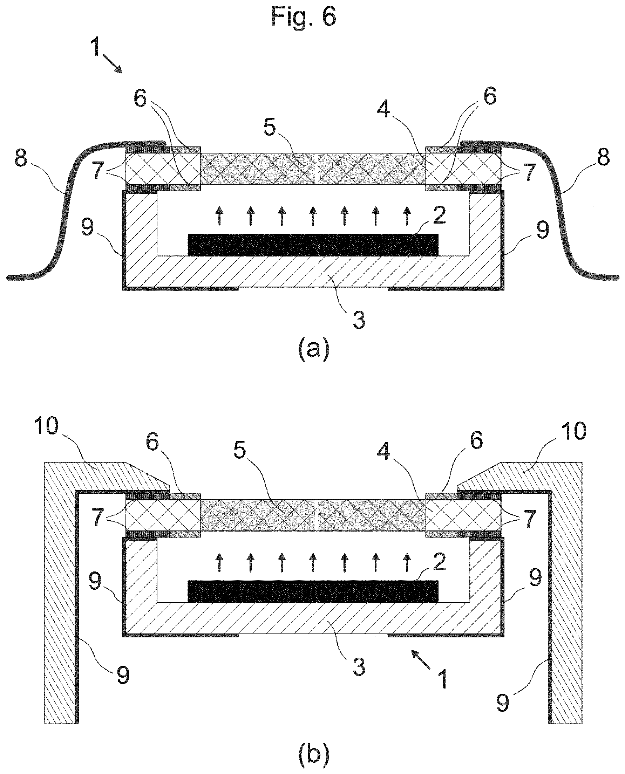

Fig. 6 Bevorzugte Ausführungsformen der erfindungsgemäßen thermischen Strahlungsquelle mit beidseitiger Anordnung eines Temperatursensors und Strahlungssensors innerhalb und außerhalb des Gehäuses im Messbereich des Austrittsfensters:

(a) Mit außenliegender Drahtkontaktierung sowie einer Kontaktierung über Kontaktflächen am Gehäuse;
(b) Mit einer Kontaktierung über Kontaktflächen am Gehäuse sowie Kontaktflächen, die auf einer Halterung ausgebildet sind, die mit der thermischen Strahlungsquelle verbunden ist;

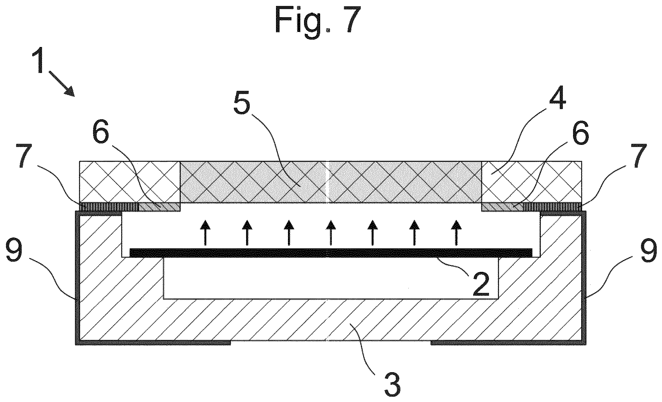

Fig. 7 Bevorzugte Ausführungsform der erfindungsgemäßen thermischen Strahlungsquelle mit einem dünnen, freitragenden Strahlungselement der thermischen Strahlungsquelle.

Figur 2 zeigt bevorzugte Ausgestaltungen des Austrittsfensters 4 der erfindungsgemäßen thermischen Strahlungsquelle 1. Das Austrittsfenster 4 der erfindungsgemäßen thermischen Strahlungsquelle 1 ist in drei verschiedene Funktionsbereiche gegliedert. Das homogene Austrittsfenster 4 weist in einer Ausführungsform bei einer Betrachtung des Querschnitts von innen nach außen einen Durchlassbereich 5, einen daran anschließenden Messbereich 6 und einen wiederum daran anschließenden Kontaktierungsbereich 7 auf. Der Durchlassbereich 5 kennzeichnet den Funktionsbereich des Austrittsfensters 4, aus dem die von dem Strahlungselement 2 der thermischen Strahlungsquelle 1 emittierte Strahlung aus dem Gehäuse 3 in die Umgebung abgestrahlt wird. Der Durchlassbereich 5 sollte vorzugsweise der Größe der strahlenden Fläche des Strahlungselementes entsprechen. Der Messbereich 6 kennzeichnet den Funktionsbereich des Austrittsfensters 4, innerhalb dem der Temperatursensor und Strahlungssensor aufgebracht und / oder integriert angeordnet ist, wobei der Messbereich 6 in einer Ausgestaltung zwischen dem Durchlassbereich 5 und dem Kontaktierungsbereich 7 ausgebildet ist. Der Kontaktierungsbereich 7 kennzeichnet den Funktionsbereich des Austrittsfensters 4, in welchem die Kontaktierung von dem Temperatursensor und Strahlungssensor erfolgt, wobei der Kontaktierungsbereich 7 im Randbereich des Austrittsfensters 4 ausgebildet ist, wo das Austrittsfenster 4 in Kontakt mit dem Gehäuse 3 der thermischen Strahlungsquelle 1 steht. Eine rechteckige oder quadratische Form des Austrittsfensters 4, wie in Fig. 2(a) gezeigt, ist insbesondere für Gehäuse der Oberflächenmontage (sog. SMD-Gehäuse), aber auch eckige Gehäuseformen der Durchsteckmontage geeignet. Ein rundes Austrittsfenster 4, wie in Fig. 2(b) gezeigt, wird vorzugsweise in runden Gehäuseformen, wie z.B. TO-Gehäusen, eingesetzt. Der Temperatursensor und Strahlungssensor ist dabei in dem Messbereich 6 auf dem Austrittsfenster 4 derart untergebracht, dass vorzugsweise ein runder oder rechteckiger Durchlassbereich 5 für das Hindurchtreten der Strahlung in der Mitte des Austrittsfensters 4 entsteht. Die Kontaktierung von Temperatursensor und Strahlungssensor erfolgt in dem Kontaktierungsbereich 7, der im Randbereich des Austrittsfensters 4 liegt, wo dieses in Kontakt mit dem Gehäuse 3 steht. Der Temperatur- und/oder Strahlungssensor ist im Messbereich 6 des Austrittsfensters 4 vorzugsweise umlaufend ausgebildet, so dass Temperatur und Strahlungsleistung exakt gemessen werden können. Es ist aber auch möglich eine Vielzahl an Temperatursensoren und Strahlungssensoren in dem Messbereich 6 des Austrittsfensters 4 anzuordnen, um eine ortsaufgelöste Temperatur- bzw. Strahlungsleistungsmessung zu realisieren.

In einem anderen Ausführungsbeispiel ist der Temperatursensor und Strahlungssensor z. B. in Form eines Platin (Pt)-Widerstandes in der Mitte des Austrittsfensters angeordnet. Die elektrische Kontaktierung erfolgt z. B. über ITO-Leiterbahnen, die vom Pt-Widerstand zum Kontaktierungsbereich am Rand des Austrittsfensters geführt werden. Ein Pt-Widerstand ist im Vergleich zum Durchlassbereich des Austrittsfensters sehr klein ausführbar, so dass er die emittierte Strahlung des Strahlungselements nicht beeinflusst. Damit kann direkt die Temperatur bzw. Strahlungsleistung in der Mitte des Austrittsfensters gemessen werden.

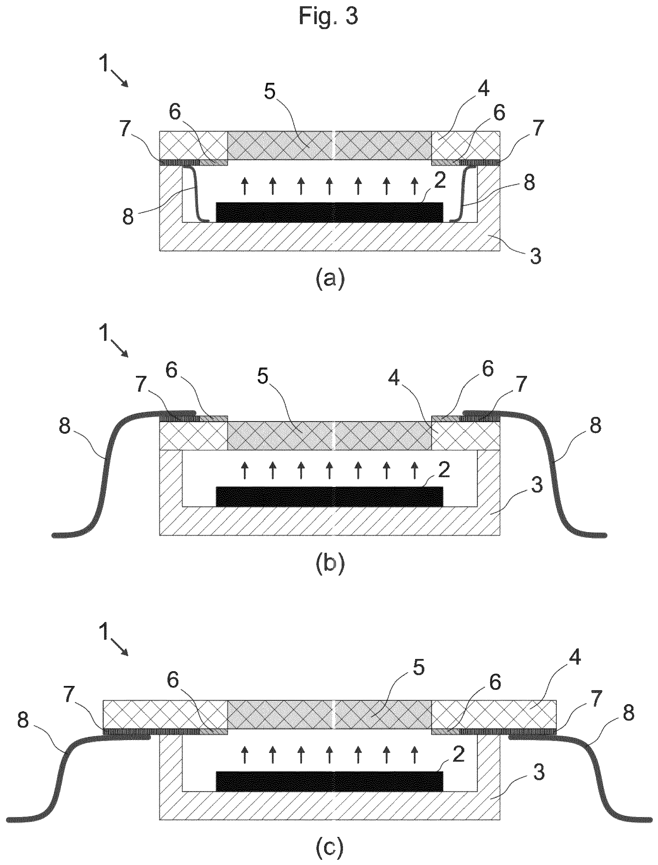

Figur 3 zeigt mögliche Ausführungsformen der erfindungsgemäßen thermischen Strahlungsquelle 1 mit im Austrittsfenster 4 aufgebrachtem Temperatursensor und Strahlungssensor im Messbereich 6 des Austrittsfensters 4 und einer Drahtkontaktierung 8 im Kontaktierungsbereich 7 des Austrittsfensters 4. Eine innenliegende Anordnung von Temperatursensor und Strahlungssensor im Messbereich 6 des Austrittsfensters 4 und deren Drahtkontaktierung 8 im Kontaktierungsbereich 7 des Austrittsfensters 4, wie in Fig. 3(a) gezeigt ist, hat u. a. den Vorteil, dass alle Komponenten vor externen Einflüssen, z. B. Beschädigungen, gut geschützt sind. Durch die innenliegende Anordnung des Temperatursensors kann dieser nicht direkt die Oberflächentemperatur der außenliegenden, einem (Mess-) Objekt zugewandten Seite des Austrittsfensters 4 messen und der Strahlungssensor kann nicht direkt die von der thermischen Strahlungsquelle 1 ausgesandte Strahlungsleistung erfassen. Beides kann aber durch eine Kalibrierung des Systems und durch Kenntnis der Materialeigenschaften, wie z. B. der Transmission und Wärmeleitfähigkeit des Austrittsfensters 4, in der Signalverarbeitung des Systems korrigiert werden.

Alternativ können der Temperatursensor und Strahlungssensor im Messbereich 6 des Austrittsfensters 4 und deren Kontaktierungsdrähte 8 im Kontaktierungsbereich 7 außerhalb des Gehäuses 3 angeordnet sein, wie in Fig. 3(b) gezeigt. In dieser Anordnung wird vorteilhafterweise direkt die von der thermischen Strahlungsquelle 1 ausgesandte Strahlungsleistung erfasst und auch die Oberflächentemperatur der einem (Mess-)Objekt zugewandten Seite des Austrittsfensters 4 gemessen. Der Temperatursensor und Strahlungssensor im Messbereich 6 des Austrittsfensters 4 und deren Kontaktdrähte 8 können durch das Aufbringen einer zusätzlichen dünnen Passivierungs- und Schutzschicht, aus z. B. Glas, vor äußeren Einflüssen geschützt werden.

Wenn bei einer innenliegenden Anordnung des Temperatursensors und Strahlungssensors im Messbereich 6 des Austrittsfensters 4 innerhalb des Gehäuses 3 kein Platz für Kontaktdrähte 8 vorhanden oder dies technologisch nicht kompatibel ist, dann kann eine außenliegende Drahtkontaktierung 8 derart erfolgen, dass das Austrittsfenster 4 über das Gehäuse 3 hinausragt und der Kontaktierungsbereich 7 ebenfalls bis nach außen reicht, wie in Fig. 3(c) gezeigt ist.

Eine besonders bevorzugte Ausgestaltung der erfindungsgemäßen thermischen Strahlungsquelle 1 ist in Figur 4 gezeigt. Um ein hochintegriertes und langzeitstabiles Bauteil für die automatisierte Herstellung in hohen Stückzahlen realisieren zu können, sind eine drahtlose Kontaktierung des Temperatursensors und Strahlungssensors, z. B. durch Kleben mit einem leitfähigen Klebstoff oder mittels Löten mit einer metallischen Lotpaste, sowie ein Gehäuse 3 für die Oberflächenmontage vorteilhaft. Die elektrische Kontaktierung des Temperatursensors und Strahlungssensors erfolgt dabei über passende Kontaktflächen 9 auf dem Gehäuse 3 für die Oberflächenmontage.

Figur 5 zeigt eine weitere bevorzugte Ausgestaltung der erfindungsgemäßen thermischen Strahlungsquelle 1 mit einer drahtlosen Kontaktierung bei einem außenliegenden, d. h. außerhalb des Gehäuses 3 angeordneten Temperatursensor und Strahlungssensors im Messbereich 6 des Austrittsfensters 4. Hierbei ist die thermische Strahlungsquelle 1 an einer Halterung 10 mit lötbaren Kontaktflächen 9 befestigt und mittels Lot oder leitfähigem Klebstoff im Kontaktierungsbereich 7 des Austrittsfensters 4 elektrisch kontaktiert. Die Halterung kann beispielsweise eine Leiterplatte oder ein anderes Gehäuse oder etwas ähnliches sein. Diese Ausgestaltung hat den Vorteil, dass die elektrischen Kontakte vor Beschädigung und anderen äußeren Umwelteinflüssen geschützt sind und nur der Messbereich 6 des Austrittsfensters 4 mit einer Passivierungs- und Schutzschicht abgedeckt werden muss.

Figur 6 zeigt eine weitere Ausgestaltung der erfindungsgemäßen thermischen Strahlungsquelle 1 mit beidseitiger Anordnung der Temperatursensoren und Strahlungssensoren auf bzw. in dem Messbereich 6 des Austrittsfensters 4. Die Kontaktierung der Sensoren kann entweder drahtlos über Kontaktflächen 9 (Fig. 6(b)), mittels Kontaktdrähten 8 oder kombiniert (Fig. 6(a)) erfolgen. Das hat den Vorteil, dass sich Temperaturunterschiede zwischen Innen- und Außenseite des Austrittsfensters 4 messen lassen, wodurch z. B. die Messgenauigkeit erhöht wird. Außerdem ist es damit möglich eine Vielzahl von Sensorelementen in dem Messbereich 6 des Austrittsfensters 4 unterzubringen, um beispielsweise die Temperatur und die Strahlungsleistung ortsaufgelöst messen zu können.

Figur 7 zeigt eine weitere bevorzugte Ausgestaltung der erfindungsgemäßen thermischen Strahlungsquelle 1, wobei das Strahlungselement bzw. Heizelement 2 eine sehr geringe thermische Masse besitzt, damit es gut elektrisch modulierbar ist, und durch einen freitragenden Aufbau eine sehr gute thermische Isolation aufweist, damit ein maximaler Wirkungsgrad erreicht wird.

Für die Messung der tatsächlichen Temperatur des Austrittsfensters kann der Temperatursensor und Strahlungssensor verwendet werden. Als Temperatursensor und Strahlungssensor wird dabei vorzugsweise ein Platin-Messwiderstand in Dünnschichttechnik verwendet. Da beide Sensoren, Temperatursensor und Strahlungssensor, in dem Messbereich des Austrittsfensters aufgebracht und / oder integriert ausgebildet sind, kann mit beiden Sensoren die Temperatur des Austrittsfenster gemessen werden.

Für die Bestimmung der abgestrahlten Strahlungsleistung stehen erfindungsgemäß zwei Verfahrensvarianten zur Verfügung.

Bei einer ersten Verfahrensvariante wird für die Bestimmung der Strahlungsleistung ein Temperatursensor und ein Strahlungssensor verwendet. Der Temperatursensor ist dafür verspiegelt ausgebildet, z. B. durch das Aufbringen einer dünnen Metallschicht. Das hat den Vorteil, dass die auf den Temperatursensor auftreffende Strahlung reflektiert und durch den Temperatursensor nicht detektiert wird, so dass der Temperatursensor nur die Temperatur des Austrittsfensters misst. Ist der Temperatursensor als ein Platin-Messwiderstand ausgebildet, dient das Platin als Spiegelschicht und erfüllt diesen Zweck. Manchmal ist es zweckdienlich, den Temperatursensor durch eine Passivierungs- und Schutzschicht zu schützen, so dass auf die Passivierungs- und Schutzschicht nochmals eine Reflexionsschicht aufgebracht werden muss, da die Passivierungs- und Schutzschicht die Strahlung unter Umständen absorbieren würde. Der Temperatursensor liefert ein erstes Sensorsignal. Der Strahlungssensor, welcher beispielsweise auch aus Platin besteht, wird hingegen hoch absorbierend für die Strahlung ausgebildet, z. B. durch das Aufbringen einer Absorptionsschicht. Der Strahlungssensor detektiert die emittierte Strahlung des thermischen Strahlungselementes, wobei ein zweites Sensorsignal erzeugt wird. Von den beiden detektierten Sensorsignalen wird eine mathematische Verknüpfung, z. B. eine Differenz, gebildet, die schließlich ein Maß für die abgestrahlte Strahlungsleistung der thermischen Strahlungsquelle ist. Mittels der weiter oben beschriebenen initialen Kalibrierung wird ein Zusammenhang zwischen der (unkalibriert) gemessenen und der tatsächlichen (wahren) Temperatur des Austrittsfenster bzw. der ausgesandten Strahlungsleistung hergestellt und in der Signalverarbeitung abgelegt und derart korrigiert, dass eine exakte Regelung ermöglicht wird.

Bei einer zweiten Verfahrensvariante wird für die Bestimmung der Strahlungsleistung ein Temperatursensor oder ein Strahlungssensor verwendet. Eine elektrische Modulation des Strahlungselementes, die eine zeitliche Strahlungsänderung bewirkt, führt zu einer zeitlichen Temperaturänderung im absorbierenden Strahlungssensor bzw. im Temperatursensor. Beide können mit einer Passivierungs- und Schutzschicht versehen sein. Diese Temperaturänderung ist detektierbar und damit die Strahlungsleistung nach einer Kalibrierung bestimmbar. Bei dieser Verfahrensvariante ist nur ein Sensorelement, entweder ein Temperatursensor oder ein Strahlungssensor, notwendig, wobei damit gleichzeitig die Messung der Temperatur des Austrittsfensters und der Strahlungsleistung möglich ist. Diese zweite Verfahrensvariante ist hinsichtlich eines einfachen und kostengünstigen Aufbaus vorteilhaft. Allerdings erfordert diese zweite Verfahrensvariante eine Kalibrierung des Systems.

**Bezugszeichenliste**

1 Thermische Strahlungsquelle
2 Strahlungselement bzw. Heizleiterelement / Heizleiterchip
3 Gehäuse der Strahlungsquelle
4 Austrittsfenster
5 Durchlassbereich des Austrittsfensters
6 Messbereich des Austrittsfensters, in dem der Temperatursensor und Strahlungssensor angeordnet sind

7 Kontaktierungsbereich des Austrittsfensters

8 Drahtkontaktierung / Kontaktdrähte

9 Kontaktfläche für Löten und/oder Kleben, drahtlose Kontaktierung

10 Halterung für thermische Strahlungsquelle

**Patentansprüche**

1. Thermische Strahlungsquelle (1) umfassend ein Gehäuse (3), ein elektrisch betriebenes Strahlungselement (2), welches in dem Gehäuse (3) angeordnet ist, sowie ein homogenes für Hautkontakt angepasstes Austrittsfenster (4), durch welches eine von dem Strahlungselement (2) emittierte elektromagnetische Strahlung aus dem Gehäuse (3) ausgesandt wird, wobei das Austrittsfenster beabstandet zum Strahlungselement angeordnet ist, wobei das Austrittsfenster (4) einen Durchlassbereich (5), einen Kontaktierungsbereich (7) sowie einen Messbereich (6) aufweist, wobei der Messbereich (6) und der Durchlassbereich (5) getrennt voneinander oder teilweise überlappend ausgebildet sind, wobei ein Temperatursensor und ein Strahlungssensor innerhalb des Messbereichs (6) des Austrittsfensters (4) angeordnet sind, der Temperatursensor ausgestattet ist zur Messung einer Temperatur des Austrittsfensters ein erstes Signal zu erzeugen und der Strahlungssensor ausgestaltet ist zur Messung der abgestrahlten Strahlungsleistung des Strahlungselements ein zweites Signal zu erzeugen, wobei der Temperatursensor und der Strahlungssensor über den Kontaktierungsbereich (7) elektrisch kontaktiert ist, wobei das Austrittsfenster (4) aus einem der Materialien Saphir oder Kalziumfluorid oder Bariumfluorid oder Zinkselenid oder Silizium oder Germanium ausgebildet ist.

2. Thermische Strahlungsquelle (1) nach Anspruch 1, **dadurch gekennzeichnet, dass** der Messbereich (6) von dem Durchlassbereich (4) und dem Kontaktierungsbereich (7) umfasst ausgebildet ist.

3. Thermische Strahlungsquelle (1) nach Anspruch 1, **dadurch gekennzeichnet, dass** der Messbereich (6) deckungsgleich mit dem Durchlassbereich (5) ausgebildet ist.

4. Thermische Strahlungsquelle (1) nach einem der Ansprüche 1 bis 3, **dadurch gekennzeichnet, dass** das Austrittsfenster (4) eine rechteckige, quadratische oder eine runde Form mit einer Dicke im Bereich von vorzugsweise 200 μm bis 2mm aufweist.

5. Thermische Strahlungsquelle (1) nach einem der Ansprüche 1 oder 2, **dadurch gekennzeichnet, dass** der Temperatursensor und der Strahlungssensor deckungsgleich mit dem Messbereich (6) des Austrittsfensters (4) ausgebildet sind.

6. Thermische Strahlungsquelle (1) nach einem der vorherigen Ansprüche, **dadurch gekennzeichnet, dass** mehr als ein Temperatursensor und mehr als ein Strahlungssensor innerhalb des Messbereichs (6) des Austrittsfensters (4) angeordnet ist.

7. Thermische Strahlungsquelle (1) nach einem der vorherigen Ansprüche, **dadurch gekennzeichnet, dass** der Temperatursensor und der Strahlungssensor als Platin-Messwiderstände ausgebildet sind.

8. Thermische Strahlungsquelle (1) nach Anspruch 7, **dadurch gekennzeichnet, dass** der Temperatursensor eine Reflexionsschicht aufweist und der Strahlungssensor eine Absorptionsschicht aufweist und / oder der Temperatursensor und der Strahlungssensor eine Schutz- und Passivierungsschicht aufweisen.

9. Thermische Strahlungsquelle (1) nach Anspruch 1, **dadurch gekennzeichnet, dass** die im Messbereich (6) des Austrittsfensters (4) aufgebrachten Temperatursensoren und Strahlungssensoren innerhalb des Gehäuses (3) angeordnet sind, wobei eine elektrische Drahtkontaktierung (8) des Temperatursensors und des Strahlungssensors ebenfalls innerhalb des Gehäuses (3) ausgebildet ist.

10. Thermische Strahlungsquelle (1) nach Anspruch 1, **dadurch gekennzeichnet, dass** die im Messbereich (6) des Austrittsfensters (4) aufgebrachten Temperatursensoren und Strahlungssensoren außerhalb des Gehäuses (3) angeordnet sind, wobei eine elektrische Drahtkontaktierung (9) des Temperatursensors und des Strahlungssensors ebenfalls außerhalb des Gehäuses (4) ausgebildet ist.

11. Thermische Strahlungsquelle (1) nach Anspruch 1, **dadurch gekennzeichnet, dass** die im Messbereich (6) des Austrittsfensters (4) aufgebrachten Temperatursensoren und Strahlungssensoren innerhalb des Gehäuses (3) angeordnet sind, wobei eine elektrische Drahtkontaktierung (9) des Temperatur- und Strahlungssensors außerhalb des Gehäuses (3) ausgebildet ist.

12. Thermische Strahlungsquelle (1) nach Anspruch 1, **dadurch gekennzeichnet, dass** eine Kontaktierung des Temperatursensors und des Strahlungssensors über innerhalb oder außerhalb an dem Gehäuse angebrachte Kontaktflächen (9) mittels Löten einer metallischen Lotpaste und / oder mittels leitfähigem Klebstoff ausgebildet ist.

13. Thermische Strahlungsquelle (1) nach Anspruch 1, **dadurch gekennzeichnet, dass** die im Messbereich (6) des Austrittsfensters (4) aufgebrachten Temperatursensoren und Strahlungssensoren außerhalb des Gehäuses (3) angeordnet sind, wobei eine elektrische Kontaktierung des Temperatursensors und des Strahlungssensors über Kontaktflächen (9), die auf einer Halterung (10), die mit der thermischen Strahlungsquelle (1) verbunden ist, ausgebildet ist.

14. Thermische Strahlungsquelle (1) nach Anspruch 1, **dadurch gekennzeichnet, dass** sowohl der Temperatursensor und der Strahlungssensor innerhalb des Gehäuses (3) im Messbereich (6) des Austrittsfensters (4) aufgebracht angeordnet sind als auch ein weiterer Temperatursensor und ein weiterer Strahlungssensor außerhalb des Gehäuses (3) im Messbereich (6) des Austrittsfensters (4) aufgebracht angeordnet sind, wobei eine elektrische Kontaktierung der Temperatursensoren und der Strahlungssensoren mittels einer Drahtkontaktierung (8) und / oder über außerhalb an dem Gehäuse (3) angebrachte Kontaktflächen (9) mittels Löten einer metallischen Lotpaste und / oder mittels leitfähigem Klebstoff ausgebildet ist.

15. Thermische Strahlungsquelle (1) nach einem der Ansprüche 1 bis 6 oder 9 bis 14, **dadurch gekennzeichnet, dass** das Austrittsfenster (4) aus Silizium oder Germanium ausgebildet ist, wobei der Temperatursensor und der Strahlungssensor im Messbereich (6) des Austrittsfensters (4) integriert angeordnet sind.

16. Thermische Strahlungsquelle (1) nach einem der vorherigen Ansprüche, **dadurch gekennzeichnet, dass** das elektrische Strahlungselement (2) freitragend ausgebildet ist, wobei unterhalb des Strahlungselementes (4) eine Reflexionsschicht ausgebildet ist.

17. Verfahren zur Messung der Temperatur eines Austrittsfensters (4) einer thermischen Strahlungsquelle (1) und der Strahlungsleistung einer von der thermischen Strahlungsquelle (1) emittierten Strahlung durch das homogene Austrittsfenster (4), wobei die thermische Strahlungsquelle (1) nach einem der Ansprüche 1 bis 14 oder 16 verwendet wird, wobei das Verfahren folgende Verfahrensschritte umfasst:

- Messen der Temperatur des Austrittsfensters (4) mittels des Temperatursensors, der in dem Messbereich (6) des Austrittsfensters (4) aufgebracht angeordnet ist, wobei das erste Sensorsignal erzeugt wird;
- Detektieren der emittierten Strahlung des Strahlungselementes (2), die durch den Strahlungssensor absorbiert wird, der in einem Messbereich (6) des Austrittsfensters (4) aufgebracht angeordnet ist, wobei ein zweites Sensorsignal erzeugt wird;
- Auswerten der Sensorsignale durch Bilden einer mathematischen Verknüpfung aus dem ersten Sensorsignal des Temperatursensors und dem zweiten Sensorsignal des Strahlungssensors unter Verwendung einer Kalibrierung, wobei die mathematische Verknüpfung die Strahlungsleistung angibt.

18. Verfahren zur Messung der Temperatur eines Austrittsfensters (4) einer thermischen Strahlungsquelle (1) mittels eines Temperatursensors und der Strahlungsleistung einer von der thermischen Strahlungsquelle (1) emittierten Strahlung durch das Austrittsfenster (4) mittels des Strahlungssensors, wobei die thermische Strahlungsquelle (1) nach einem der Ansprüche 1 bis 14 oder 16 verwendet wird, wobei als Sensorelement entweder der Temperatursensor oder der Strahlungssensor verwendet und mittels einer elektrischen Modulation des Strahlungselementes (2) der thermischen Strahlungsquelle (1) eine zeitliche Strahlungsänderung erzeugt wird, die eine zeitliche Temperaturänderung im verwendeten Sensorelement bewirkt und wobei daraus die Strahlungsleistung mittels einer Kalibrierung ermittelt wird.

**Claims**

1. Thermal radiation source (1) comprising a housing (3), an electrically operated radiation element (2) arranged in the housing (3), and a homogeneous exit window (4) designed for skin contact, through which electromagnetic radiation emitted by the radiation element (2) is radiated from the housing (3), wherein the exit window is spaced apart from the radiation element, wherein the exit window (4) has a transmission region (5), a contact region (7), and a measuring region (6), wherein the measuring region (6) and the transmission region (5) are separate from one another or partially overlap, wherein a temperature sensor and a radiation sensor are arranged inside the measuring region (6) of the exit window (4), the temperature sensor is designed to generate a first signal for measuring a temperature of the exit window, and the radiation sensor is designed to generate a second signal for measuring the radiated radiant power of the radiation element, wherein the temperature sensor and the radiation sensor are electrically contacted via the contact region (7), wherein the exit window (4) is made of one of the materials sapphire or calcium fluoride or barium fluoride or zinc selenide or silicon or germanium.

2. Thermal radiation source (1) according to claim 1, **characterized in that** the measuring region (6) is bordered by the transmission region (4) and the contact region (7).

3. Thermal radiation source (1) according to claim 1, **characterized in that** the measuring region (6) is congruent with the transmission region (5).

4. Thermal radiation source (1) according to any of claims 1 to 3, **characterized in that** the exit window (4) is a rectangular, square or round shape having a thickness in the range from preferably 200 $\mu$m to 2 mm.

5. Thermal radiation source (1) according to either claim 1 or claim 2, **characterized in that** the temperature sensor and the radiation sensor are congruent with the measuring region (6) of the exit window (4).

6. Thermal radiation source (1) according to any of the preceding claims, **characterized in that** more than one temperature sensor and more than one radiation sensor are arranged inside the measuring region (6) of the exit window (4).

7. Thermal radiation source (1) according to any of the preceding claims, **characterized in that** the temperature sensor and the radiation sensor are platinum resistors.

8. Thermal radiation source (1) according to claim 7, **characterized in that** the temperature sensor has a reflective layer and the radiation sensor has an absorbing layer and/or the temperature sensor and the radiation sensor have a protecting and passivating layer.

9. Thermal radiation source (1) according to claim 1, **characterized in that** the temperature sensors and radiation sensors attached in the measuring region (6) of the exit window (4) are arranged inside the housing (3), an electrical wire contact (8) of the temperature sensor and of the radiation sensor also being formed inside the housing (3).

10. Thermal radiation source (1) according to claim 1, **characterized in that** the temperature sensors and radiation sensors attached in the measuring region (6) of the exit window (4) are arranged outside the housing (3), an electrical wire contact (9) of the temperature sensor and of the radiation sensor also being formed outside the housing (4).

11. Thermal radiation source (1) according to claim 1, **characterized in that** the temperature sensors and radiation sensors attached in the measuring region (6) of the exit window (4) are arranged inside the housing (3), an electrical wire contact (9) of the temperature and radiation sensor being formed outside the housing (3).

12. Thermal radiation source (1) according to claim 1, **characterized in that** a contact of the temperature sensor and of the radiation sensor is formed using contact surfaces (9) attached to the housing on the inside or outside, by means of soldering a metal soldering paste and/or by means of conductive adhesive.

13. Thermal radiation source (1) according to claim 1, **characterized in that** the temperature sensors and radiation sensors attached in the measuring region (6) of the exit window (4) are arranged outside the housing (3), whereas an electrical contact of the temperature sensor and of the radiation sensor being formed using contact surfaces (9) is formed on a support (10) which is connected to the thermal radiation source (1).

14. Thermal radiation source (1) according to claim 1, **characterized in that** the temperature sensor and the radiation sensor are attached inside the housing (3) in the measuring region (6) of the exit window (4), and a further temperature sensor and a further radiation sensor are attached outside the housing (3) in the measuring region (6) of the exit window (4), an electrical contact of the temperature sensors and of the radiation sensors being formed by means of a wire contact (8) and/or using contact surfaces (9) attached to the housing (3) on the outside, by means of soldering a metal soldering paste and/or by means of conductive adhesive.

15. Thermal radiation source (1) according to any of claims 1 to 6 or 9 to 14, **characterized in that** the exit window (4) is made of silicon or germanium, the temperature sensor and the radiation sensor being integrated in the measuring region (6) of the exit window (4).

16. Thermal radiation source (1) according to any of the preceding claims, **characterized in that** the electrical radiation element (2) is self-supporting, a reflective layer being formed below the radiation element (4).

17. Method for measuring the temperature of an exit window (4) of a thermal radiation source (1) and for measuring the radiant power of radiation emitted by the thermal radiation source (1) through the homogeneous exit window (4), wherein the thermal radiation source (1) according to any of claims 1 to 14 or 16 is used, wherein the method comprises the method steps of:

- measuring the temperature of the exit window (4) by means of the temperature sensor, which is attached in the measuring region (6) of the exit window (4), wherein the first sensor signal is generated;

- detecting the emitted radiation of the radiation element (2) which is absorbed by the radiation sensor, which is attached in a measuring region (6) of the exit window (4), wherein a second sensor signal is generated;

- evaluating the sensor signals by forming a mathematical operation from the first sensor signal of the temperature sensor and the second sensor signal of the radiation sensor using a calibration, wherein the mathematical operation indicates the radiant power.

18. Method for measuring the temperature of an exit window (4) of a thermal radiation source (1) by means of a temperature sensor, and for measuring the radiant power of radiation, emitted by the thermal radiation source (1) through the exit window (4), by means of the radiation sensor, wherein the thermal radiation source (1) according to any of claims 1 to 14 or 16 is used, wherein either the temperature sensor or the radiation sensor is used as a sensor element, and a radiation change over time is generated by means of an electrical modulation of the radiation element (2) of the thermal radiation source (1), which change causes a temperature change over time in the sensor element used, and wherein the radiant power is ascertained therefrom by means of a calibration.

**Revendications**

1. Source de rayonnement thermique (1) comprenant un boîtier (3), un élément de rayonnement (2) actionné électriquement, lequel est disposé dans le boîtier (3), ainsi qu'une fenêtre de sortie (4) homogène adaptée au contact avec la peau, à travers laquelle un rayonnement électromagnétique émis par l'élément de rayonnement (2) est émis à partir du boîtier (3), la fenêtre de sortie étant disposée à distance de l'élément de rayonnement, la fenêtre de sortie (4) présentant une zone de transmission (5), une zone de contact (7) ainsi qu'une zone de mesure (6), la zone de mesure (6) et la zone de transmission (5) étant réalisées séparément l'une de l'autre ou de manière à se chevaucher partiellement, un capteur de température et un capteur de rayonnement étant disposés à l'intérieur de la zone de mesure (6) de la fenêtre de sortie (4), le capteur de température étant équipé pour produire un premier signal pour la mesure d'une température de la fenêtre de sortie et le capteur de rayonnement étant conçu pour produire un second signal pour la mesure de la puissance de rayonnement émise par l'élément de rayonnement, le capteur de température et le capteur de rayonnement étant mis en contact électrique par l'intermédiaire de la zone de contact (7), la fenêtre de sortie (4) étant réalisée à partir de l'un des matériaux que sont le saphir ou le fluorure de calcium ou le fluorure de baryum ou le séléniure de zinc ou le silicium ou le germanium.

2. Source de rayonnement thermique (1) selon la revendication 1, **caractérisée en ce que** la zone de mesure (6) est réalisée par la zone de transmission (4) et la zone de contact (7).

3. Source de rayonnement thermique (1) selon la revendication 1, **caractérisée en ce que** la zone de mesure (6) est réalisée de manière à coïncider avec la zone de transmission (5).

4. Source de rayonnement thermique (1) selon l'une quelconque des revendications 1 à 3, **caractérisée en ce que** la fenêtre de sortie (4) présente une forme rectangulaire, carrée ou circulaire avec une épaisseur comprise dans une plage de préférence de 200 μm à 2 mm.

5. Source de rayonnement thermique (1) selon l'une des revendications 1 ou 2, **caractérisée en ce que** le capteur de température et le capteur de rayonnement sont réalisés de manière à coïncider avec la zone de mesure (6) de la fenêtre de sortie (4).

6. Source de rayonnement thermique (1) selon l'une des revendications précédentes, **caractérisée en ce que** plus d'un capteur de température et plus d'un capteur de rayonnement sont disposés à l'intérieur de la zone de mesure (6) de la fenêtre de sortie (4).

7. Source de rayonnement thermique (1) selon l'une des revendications précédentes, **caractérisée en ce que** le capteur de température et le capteur de rayonnement sont réalisés sous forme de résistances de mesure en platine.

8. Source de rayonnement thermique (1) selon la revendication 7, **caractérisée en ce que** le capteur de température présente une couche de réflexion et le capteur de rayonnement présente une couche d'absorption et/ou le capteur de température et le capteur de rayonnement présentent une couche de protection et de passivation.

9. Source de rayonnement thermique (1) selon la revendication 1, **caractérisée en ce que** les capteurs de température et les capteurs de rayonnement appliqués dans la zone de mesure (6) de la fenêtre de sortie (4) sont disposés à l'intérieur du boîtier (3), une mise en contact par fil (8) électrique du capteur de température et du capteur de rayonnement étant également réalisée à l'intérieur du boîtier (3).

10. Source de rayonnement thermique (1) selon la revendication 1, **caractérisée en ce que** les capteurs de température et les capteurs de rayonnement appliqués dans la zone de mesure (6) de la fenêtre de sortie (4) sont disposés à l'extérieur du boîtier (3), une mise en contact par fil électrique (9) du capteur de température et du capteur de rayonnement étant également réalisée à l'extérieur du boîtier (4).

11. Source de rayonnement thermique (1) selon la revendication 1, **caractérisée en ce que** les capteurs de température et les capteurs de rayonnement appliqués dans la zone de mesure (6) de la fenêtre de sortie (4) sont disposés à l'intérieur du boîtier (3), une mise en contact par fil électrique (9) du capteur de température et du capteur de rayonnement étant réalisée à l'extérieur du boîtier (3).

12. Source de rayonnement thermique (1) selon la revendication 1, **caractérisée en ce qu'**une mise en contact du capteur de température et du capteur de rayonnement par l'intermédiaire de surfaces de contact (9) appliquées sur le boîtier à l'intérieur ou à l'extérieur de celui-ci est réalisée par le biais d'un brasage d'une pâte à braser métallique et/ou par le biais d'un adhésif conducteur.

13. Source de rayonnement thermique (1) selon la revendication 1, **caractérisée en ce que** les capteurs de température et les capteurs de rayonnement appliqués dans la zone de mesure (6) de la fenêtre de sortie (4) sont disposés à l'extérieur du boîtier (3), une mise en contact électrique du capteur de température et du capteur de rayonnement étant réalisée par l'intermédiaire de surfaces de contact (9) qui sur un support (10) qui est relié à la source de rayonnement thermique (1).

14. Source de rayonnement thermique (1) selon la revendication 1, **caractérisée en ce qu'**à la fois le capteur de température et le capteur de rayonnement sont disposés de manière à être appliqués à l'intérieur du boîtier (3) dans la zone de mesure (6) de la fenêtre de sortie (4) et un autre capteur de température et un autre capteur de rayonnement sont disposés de manière à être appliqués à l'extérieur du boîtier (3) dans la zone de mesure (6) de la fenêtre de sortie (4), une mise en contact électrique des capteurs de température et des capteurs de rayonnement étant réalisée par le biais d'une mise en contact par fil (8) et/ou par l'intermédiaire de surfaces de contact (9) appliquées sur le boîtier (3) à l'extérieur de celui-ci par le biais d'un brasage d'une pâte à braser métallique et/ou par le biais d'un adhésif conducteur.

15. Source de rayonnement thermique (1) selon l'une des revendications 1 à 6 ou 9 à 14, **caractérisée en ce que** la fenêtre de sortie (4) est réalisée à partir de silicium ou de germanium, le capteur de température et le capteur de rayonnement étant disposés de manière à être intégrés dans la zone de mesure (6) de la fenêtre de sortie (4).

16. Source de rayonnement thermique (1) selon l'une des revendications précédentes, **caractérisée en ce que** l'élément de rayonnement (2) électrique est réalisé en porte-à-faux, une couche de réflexion étant réalisée au-dessous de l'élément de rayonnement (4).

17. Procédé permettant de mesurer la température d'une fenêtre de sortie (4) d'une source de rayonnement thermique (1) et la puissance de rayonnement d'un rayonnement émis par la source de rayonnement thermique (1) à travers la fenêtre de sortie (4) homogène, la source de rayonnement thermique (1) étant utilisée selon l'une des revendications 1 à 14 ou 16, le procédé comprenant les étapes de procédé suivantes :

   - mesure de la température de la fenêtre de sortie (4) par le biais du capteur de température qui est disposé de manière à être appliqué dans la zone de mesure (6) de la fenêtre de sortie (4), le premier signal de capteur étant produit ;
   - détection du rayonnement émis par l'élément de rayonnement (2) qui est absorbé par le capteur de rayonnement qui est disposé de manière à être appliqué dans une zone de mesure (6) de la fenêtre de sortie (4), un second signal de capteur étant produit ;
   - évaluation des signaux de capteur par formation d'une combinaison mathématique à partir du premier signal de capteur du capteur de température et du second signal de capteur du capteur de rayonnement à l'aide d'un étalonnage, la combinaison mathématique indiquant la puissance de rayonnement.

18. Procédé permettant de mesurer la température d'une fenêtre de sortie (4) d'une source de rayonnement thermique (1) par le biais d'un capteur de température et de la puissance de rayonnement d'un rayonnement émis par la source de rayonnement thermique (1) à travers la fenêtre de sortie (4) par le biais du capteur de rayonnement, la source de rayonnement thermique (1) étant utilisée selon l'une des revendications 1 à 14 ou 16, soit le capteur de température soit le capteur de rayonnement étant utilisé comme élément de détection et une variation temporelle de rayonnement qui provoque une variation temporelle de température dans l'élément de détection utilisé étant produite par le biais d'une modulation électrique de l'élément de rayonnement (2) de la source de rayonnement thermique (1) et la puissance de rayonnement étant déterminée par le biais d'un étalonnage à partir de celle-ci.

# Fig. 1

# Fig. 2

(a)

(b)

# Fig. 3

(a)

(b)

(c)

# Fig. 4

# Fig. 5

## Fig. 6

(a)

(b)

# Fig. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5391875 A
- US 8859303 B2
- US 20160361449 A1
- DE 19807453 A1
- DE 102019109842 A1
- US 2004133084 A1